# EUROPEAN PATENT APPLICATION

(11) **EP 4 796 641 A1**
(43) Date of publication of application: **26.08.2026**
(21) Application number: 24878646.9
(22) Date of filing: 13.08.2024
(51) Int. Cl.: C12N 15/864, C12N 15/867, C12N 15/861, C12N 15/62, A61K 48/00, A61P 1/16, A61P 31/20

(54) **VIRAL GENE THERAPY VECTOR FOR REMOVING HEPATITIS B VIRUSES AND USE**

(30) Priority: 19.10.2023 CN 202311358227
(71) Applicant: GUANGZHOU CHASER BIOTECHNOLOGY CO., LTD., Guangdong 511455 (CN)
(72) Inventor: SHI, Ming, Harbin, Heilongjiang 150001 (CN); REN, Haoran, Harbin, Heilongjiang 150001 (CN); CHEN, Yuehang, Harbin, Heilongjiang 150001 (CN); LI, Ying, Harbin, Heilongjiang 150001 (CN); FENG, Jia, Harbin, Heilongjiang 150001 (CN); MA, Huihui, Harbin, Heilongjiang 150001 (CN); GAO, Da, Harbin, Heilongjiang 150001 (CN)
(74) Representative: Michalski Hüttermann & Partner mbB
(86) International application number: PCT/CN2024/111713
(87) International publication number: WO 2025/081994

(57) **Abstract**

The present invention relates to a viral gene therapy vector for removing hepatitis B viruses, a preparation method therefor, and the use thereof, aiming to solve the problem in which existing viral gene therapy vectors can only inhibit the replication of hepatitis B viruses, but cannot remove virus cccDNA. The vector comprises a promoter, an intron, an enhancer, a nuclear localization signal (NLS) functional domain encoding gene of PGLYRP2 protein, an HBV DNA binding domain encoding gene of the PGLYRP2 protein, a secretory signal peptide functional domain encoding gene of the PGLYRP2 protein, an HBV nucleocapsid-binding functional domain encoding gene of the PGLYRP2 protein, and an IRES fragment. The viral gene therapy vector can remarkably promote the removal of HBVs in the liver of mice and is used for treatment of hepatitis B virus infection.

## Description

### TECHNICAL FIELD

The present disclosure relates to the field of biomedical technology, in particular to a gene therapy vector, a preparation method therefor, and the use thereof.

### BACKGROUND

Adeno-associated virus (AAV) is a single-stranded virus with an icosahedral structure. Recombinant AAV (rAAV) consists of the same capsid sequence and structure as found in wild-type adeno-associated virus. Compared to other viral delivery systems, rAAV has advantages such as long-lasting expression levels, extremely low immunogenicity, a broad range of host infection, strong infection spreading capability, and high specificity. However, there are still many issues in clinical applications. For example, the exogenous gene of rAAV is overexpressed in the liver, causing the expression level of the target protein to exceed the physiological safety threshold of the body, resulting in relatively severe liver toxicity. How to achieve long-lasting and controllable expression of rAAV within the safety threshold is a key problem to be solved in the clinical application of rAAV.

Hepatitis B virus (HBV) is the pathogen that causes hepatitis B, which belongs to the hepadnavirus family. HBV primarily infects human hepatocytes, and its infection can significantly increase the incidence of liver cirrhosis and liver cancer. For HBV-infected individuals, the main clinical treatments include interferon-alpha and nucleoside analogs, or polyclonal antibodies targeting its surface antigen. Patent CN101906417A discloses a recombinant adeno-associated virus gene therapy vector, which uses genetic recombination technology to clone shRNA that inhibits hepatitis B virus into the backbone plasmid of the adeno-associated viral vector, and co-transfects packaging cells with helper plasmids to obtain the recombinant adeno-associated virus. Although the recombinant adeno-associated virus can effectively inhibit the replication and expression of hepatitis B virus, it cannot eliminate viral cccDNA and can only slow down the disease progression, leading to a high likelihood of recurrence after discontinuation of treatment.

### SUMMARY

The present disclosure aims to solve the problem in which existing viral gene therapy vectors can only inhibit the replication of hepatitis B virus but cannot eliminate viral cccDNA, and provides a viral gene therapy vector for eliminating hepatitis B virus and the use thereof.

The present disclosure provides a viral gene therapy vector for eliminating hepatitis B virus, comprising a promoter, an intron, an enhancer, an encoding gene of the nuclear localization signal (NLS) functional domain of the PGLYRP2 protein, an encoding gene of the HBV DNA-binding domain of the PGLYRP2 protein, an encoding gene of the secretory signal peptide functional domain of the PGLYRP2 protein, an encoding gene of the HBV nucleocapsid-binding functional domain of the PGLYRP2 protein, and an IRES fragment.

The promoter is a truncated promoter M of the PGLYRP2 gene, the nucleotide sequence of which is shown in SEQ ID NO: 1 in the sequence listing.

The enhancer comprises a CMV enhancer and an HBV EnII element, wherein the nucleotide sequence of the CMV enhancer is shown in SEQ ID NO: 2 in the sequence listing, and the nucleotide sequence of the HBV EnII element is shown in SEQ ID NO: 3 in the sequence listing.

The encoding gene of the nuclear localization signal (NLS) functional domain of the PGLYRP2 protein is a gene encoding amino acids 550 to 576 at the C-terminus of the PGLYRP2 protein, the nucleotide sequence of which is shown in SEQ ID NO: 4 in the sequence listing.

The nucleotide sequence of the encoding gene of the HBV DNA-binding domain of the PGLYRP2 protein is shown in SEQ ID NO: 5 in the sequence listing.

The nucleotide sequence of the encoding gene of the secretory signal peptide functional domain of the PGLYRP2 protein is shown in SEQ ID NO: 6 in the sequence listing.

The nucleotide sequence of the encoding gene of the HBV nucleocapsid-binding functional domain of the PGLYRP2 protein is shown in SEQ ID NO: 7 in the sequence listing.

Further, the vector backbone of the viral gene therapy vector is an adeno-associated viral vector, an adenoviral vector, a lentiviral vector, or a retroviral vector; the adeno-associated viral vector is an AAV2, AAV5, AAV7, AAV8, AAV9, or AAV-DJ adeno-associated viral vector.

The present disclosure further provides the use of the viral gene therapy vector described above in the manufacture of a medicament for treating hepatitis B virus infection.

### Beneficial effects of the present disclosure:

The viral gene therapy vector of the present disclosure comprises a gene therapy delivery vector pAAV-EnII-EnCMV-M. The gene therapy delivery vector pAAV-EnII-EnCMV-M constructed in the present disclosure exhibits long-lasting expression and liver-specific expression in mice and possesses a self-feedback mechanism, endowing the gene therapy product with improved safety.

The present disclosure identifies four functional domains of the PGLYRP2 protein, namely, the HBV DNA-binding domain, nuclear localization signal (NLS) functional domain, secretory signal peptide (SP) functional domain, and nucleocapsid-binding functional domain of PGLYRP2, which are functionally combined to form two novel fusion proteins. The viral gene therapy vector of the present disclosure can simultaneously express two fusion proteins in hepatocytes: a fusion protein comprising the HBV DNA-binding domain and nuclear localization signal (NLS) functional domain of PGLYRP2 (PGLYRP2^{209-377aa}-NLS), and a fusion protein comprising the secretory signal peptide (SP) functional domain and nucleocapsid-binding functional domain of PGLYRP2 (SP-PGLYRP2^{PGRP}). These two fusion proteins respectively target HBV cccDNA and chromosomally integrated DNA as well as HBV nucleocapsids, functioning as a transcription inhibitor of HBV cccDNA and chromosomally integrated DNA and as an HBV capsid assembly modulator, which achieves a dual mechanism and synergistic effect for elimination of HBV in host cells, and can significantly promote elimination of HBV in the liver of mice. The viral gene therapy vector of the present disclosure can significantly eliminate cccDNA within 14 weeks, indicating that the method has great potential to achieve a complete cure for hepatitis B.

### BRIEF DESCRIPTION OF THE DRAWINGS

FIG. 1 is a schematic diagram of the vector pAAV-EnII-EnCMV-M;
FIG. 2 shows identification of the enhancement of vector expression by the promoter and enhancer EnII-EnCMV-M;
FIG. 3 shows specific high expression of the gene therapy delivery vector pAAV-EnII-EnCMV-M in hepatocytes;
FIG. 4 shows the positive regulatory effect of the PGLYRP2 protein on the backbone vector pAAV-EnCMV-M;
FIG. 5 shows the self-feedback regulatory effect of the PGLYRP2 protein on the gene therapy delivery vector pAAV-EnII-EnCMV-M;
FIG. 6 shows the silver staining results of rAAV-EnII-EnCMV-M-Luciferase virus;
FIG. 7 shows the relative luciferase activity of different cells infected with rAAV-EnII-EnCMV-M-Luciferase virus;
FIG. 8 shows the *in vivo* imaging results of mice injected with rAAV-EnII-EnCMV-M-Luciferase virus via the tail vein;
FIG. 9 shows the nuclear localization of the PGLYRP2 protein by confocal microscopy;
FIG. 10 shows analysis of the function of the nuclear localization signal (NLS) functional domain of the PGLYRP2 protein by confocal microscopy;
FIG. 11 shows analysis of the function of the secretory signal peptide (SP) functional domain of the PGLYRP2 protein by confocal microscopy;
FIG. 12 is a flowchart of Flag pull-down for cell culture supernatant;
FIG. 13 shows the results of Flag pull-down from cell culture supernatant;
FIG. 14 shows identification of the HBV DNA-binding domain of PGLYRP2 by HBV DNA-strep pull-down;
FIG. 15 shows analysis of the HBV replication inhibitory function of the HBV DNA-binding domain PGLYRP2²⁰⁹⁻³⁷⁷ of PGLYRP2;
FIG. 16 shows detection of the binding between the PGLYRP2 protein and the HBV nucleocapsid by co-immunoprecipitation and native polyacrylamide gel electrophoresis;
FIG. 17 shows analysis of the interaction between the PGLYRP2^{PGRP} functional domain of the PGLYRP2 protein and the HBV core antigen by co-immunoprecipitation;
FIG. 18 is a schematic diagram of the four functional domains of PGLYRP2 identified in the present disclosure;
FIG. 19 is a schematic diagram of the adeno-associated virus gene therapy vector of the present disclosure;
FIG. 20 shows the HBV clearance effect of the adeno-associated virus gene therapy vector on an infection model in mice;
FIG. 21 shows expression of the target gene in the liver of mice 14 weeks after injection of the packaged virus from the adeno-associated virus gene therapy vector via the tail vein;
FIG. 22 shows relative levels of HBV cccDNA in the liver of mice 14 weeks after injection of the packaged virus from the adeno-associated virus gene therapy vector via the tail vein;
FIG. 23 shows HBsAg levels in the serum of mice 14 weeks after injection of the packaged virus from the adeno-associated virus gene therapy vector via the tail vein.

### DETAILED DESCRIPTION

The examples of the present disclosure are described in detail below. The following examples are implemented based on the technical solution of the present disclosure, providing detailed embodiments and specific operations. However, the protection scope of the present disclosure is not limited to the following examples.

The present disclosure first constructs the gene therapy delivery vector pAAV-EnII-EnCMV-M, which is a gene therapy vector featuring liver-specific expression and a self-feedback mechanism that inhibits overexpression. Subsequently, based on the above, an adeno-associated virus gene therapy vector is further constructed, which expresses four functional domains of PGLYRP2, namely a fusion protein comprising the HBV DNA-binding domain and nuclear localization signal (NLS) functional domain of PGLYRP2 (functioning as a transcription inhibitor of HBV cccDNA and chromosomally integrated DNA), and a fusion protein comprising the secretory signal peptide (SP) functional domain and nucleocapsid-binding functional domain of PGLYRP2 (functioning as an HBV capsid assembly modulator). The tissue specificity and safety of the adeno-associated virus gene therapy vector are achieved through specific interactions between the vector and the expression products of the PGLYRP2 gene or its truncated functional domains. The gene therapy drug can achieve long-lasting, specific, and controllable expression of fusion proteins comprising truncated functional domains of PGLYRP2 in HBV-infected hepatocytes, which respectively target HBV cccDNA and HBV nucleocapsids, and synergistically function as an HBV cccDNA inhibitor and an HBV capsid assembly modulator.

The PGLYRP2 gene is constitutively expressed at high levels in normal human hepatocytes, whereas its expression is relatively low in hepatocytes with high HBV indices. The present disclosure employs partial PGLYRP2 gene promoter elements and PGLYRP2-regulated self-feedback elements to target hepatocytes for self-feedback expression of fusion proteins comprising functional domains of PGLYRP2, thereby restoring a moderately elevated level of functional domains of PGLYRP2 in HBV-infected hepatocytes. Accordingly, the present disclosure theoretically possesses safety.

### Example 1: Construction of the gene therapy delivery vector pAAV-EnII-EnCMV-M and identification of expression

Using BamHI-luciferase F and HindIII-luciferase R as primers, the Luciferase gene was amplified from the plasmid PGL4-PGLYRP2 (1--2005) as the template. The forward primer BamHI-luciferase F was CGGGATCCCGATGGAAGACGCCAAAAACATAAAGAAA, and the reverse primer HindIII-luciferase R was CCAAGCTTGGTTACACGGCGATCTTTCCGCCCTTC. The nucleotide sequence of the Luciferase gene is shown in SEQ ID NO: 9 in the sequence listing.

Using MluI-M F and NcoI-M R as primers, the truncated promoter M of the PGLYRP2 gene was amplified from the plasmid PGL4-PGLYRP2 (1--2005) as the template. The forward primer MluI-M F was CGACGCGTCGGTGGCGCATGCCTGTAACCTGA, and the reverse primer NcoI-M R was CATGCCATGGCATGGATTTCAAGCCACCAGCAGTAGCTG. The nucleotide sequence of the truncated promoter M of the PGLYRP2 gene is shown in SEQ ID NO: 1 in the sequence listing.

The plasmid PGL4-PGLYRP2 (1--2005), used for amplification of the Luciferase gene and the truncated promoter M of the PGLYRP2 gene, has been disclosed in the article Hepatology. 2020 May; 71(5): 1626-1642.

Using MluI-HBV-EnII F and SacI-HBV-EnII R as primers, the HBV EnII element was amplified from the plasmid pBB4.5-HBV1.2, genotype C, as the template. The forward primer MluI-HBV-EnII F was CGACGCGTCGTCCTGCCCAAGGTCTTACATAA, and the reverse primer SacI-HBV-EnII R was CGAGCTCGCAGCTCCTCCCAGTCCTTAAAC. The nucleotide sequence of the HBV EnII element is shown in SEQ ID NO: 3 in the sequence listing.

The plasmid pBB4.5-HBV1.2, genotype C, used for amplification of the HBV EnII element, was gifted by others and has been disclosed in the article Emerg Microbes Infect. 2022 Dec; 11(1): 1356-1370. The AAV-MCS empty vector was purchased from MiaoLing Plasmid Platform (P0244, containing a CMV promoter).

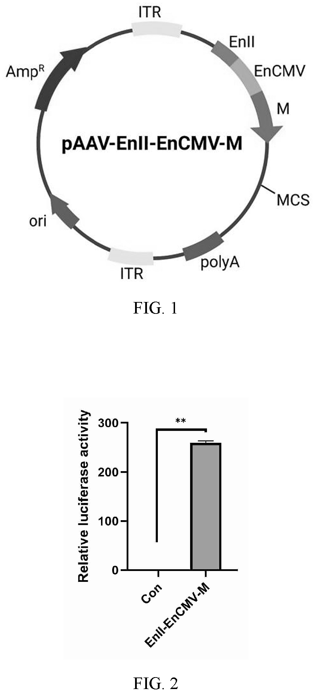

The AAV-MCS empty vector was double-digested with the restriction enzymes MluI and NcoI to remove the CMV promoter while retaining the CMV enhancer, and ligated with the truncated promoter M of the PGLYRP2 gene to obtain pAAV-EnCMV-M. The nucleotide sequence of EnCMV is shown in SEQ ID NO: 2 in the sequence listing. Subsequently, pAAV-EnCMV-M was double-digested with the restriction enzymes MluI and SacI, and ligated with the HBV EnII element to finally construct the vector pAAV-EnII-EnCMV-M. A schematic diagram of the vector is shown in FIG. 1.

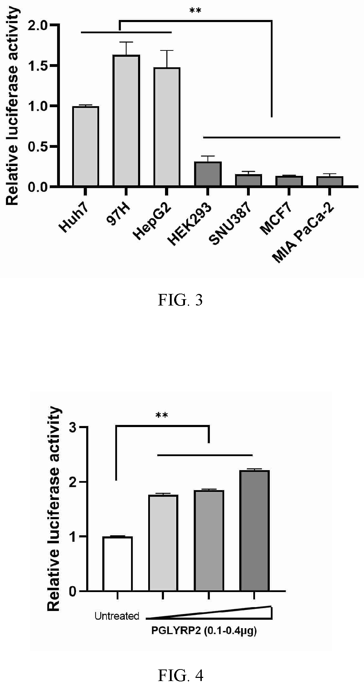

After sequence verification of the inserted fragments, the gene therapy delivery vector pAAV-EnII-EnCMV-M was double-digested with the restriction enzymes BamHI and HindIII, and ligated with the Luciferase gene sequence to obtain the recombinant plasmid pAAV-EnII-EnCMV-M-Luciferase, which was used for identification of the expression level of the gene therapy delivery vector. Equal molar amounts of the control plasmid pAAV-Basic-Luciferase (Con group) or the recombinant plasmid pAAV-EnII-EnCMV-M-Luciferase were transfected into the hepatocyte cell line Huh7. After 12 hours of transfection, cells were lysed, and luciferase substrate was added to the cell lysate at a 1:1 volume ratio. The luciferase intensity was measured using a fluorometer. Luciferase reporter gene assay indicated that the pAAV-EnII-EnCMV-M-Luciferase group exhibited significantly higher luciferase activity in Huh7 hepatocytes compared to the control pAAV-Basic-Luciferase (Con group) with no promoter or enhancer (as shown in FIG. 2). Moreover, equal doses of the recombinant plasmid pAAV-EnII-EnCMV-M-Luciferase and a Renilla luciferase plasmid was transfected into cells derived from different tissues. After 12 hours of transfection, cells were lysed, and luciferase substrate was added to the cell lysate at a 1:1 volume ratio. The luciferase intensity was measured using a fluorometer. The results demonstrated that pAAV-EnII-EnCMV-M-Luciferase exhibited higher expression specificity in hepatocytes than in non-hepatocytes (as shown in FIG. 3).

### Example 2: Analysis of hepatocyte-specific expression and self-feedback regulation of the gene therapy delivery vector pAAV-EnII-EnCMV-M by PGLYRP2 protein

First, the gene therapy delivery vectors pAAV-EnII-EnCMV-M-Luciferase and pAAV-EnCMV-M-Luciferase were cultured overnight with shaking, followed by plasmid extraction (according to the kit instructions, Omega, D6943-02). The obtained plasmids were purified as follows: 1/10 volume of sodium acetate (3 M, pH 5.2) and 7/10 volume of isopropanol were added to the extracted plasmids, mixed thoroughly, and allowed to stand at room temperature for 5 minutes, followed by centrifugation at 12,000 rpm for 10 minutes. A white DNA precipitate was visible. 1 mL of 70% ethanol was then added, followed by centrifugation at 12,000 rpm for 10 minutes. The supernatant was discarded, residual ethanol was air-dried, and approximately 500 µL of physiological saline was added to dissolve the DNA. The DNA concentration was measured and recorded. The plasmid pLVSIN-PGLYRP2 used for co-transfection has been disclosed in the article Hepatology. 2020 May; 71(5): 1626-1642. Huh7 cells cultured in a 12-well cell culture plate were divided into two groups, designated as Group 1 and Group 2. Each group included three experimental wells and one control well. Group 1 was transfected with 0.5 µg of pAAV-EnII-EnCMV-M-Luciferase per well, and Group 2 was transfected with 0.5 µg of pAAV-EnCMV-M-Luciferase per well. The control well for each group was transfected with 0.5 µg of the PLVSIN empty vector (purchased from TAKARA). The experimental wells for each group were transfected with 0.1 µg, 0.2 µg, and 0.4 µg of the plasmid pLVSIN-PGLYRP2, respectively. After 24 hours, the luciferase intensity was measured using a luciferase reporter gene assay kit.

Results: As shown in FIG. 4, the PGLYRP2 protein promoted the protein expression activity of the backbone vector pAAV-EnCMV-M-Luciferase in a dose-dependent manner, and even low doses of the PGLYRP2 protein were sufficient to exhibit a certain expression-promoting effect on the backbone vector. Considering that the PGLYRP2 protein exhibits hepatocyte-specific expression characteristics, these results indicate that the vector backbone possesses hepatocyte-specific expression capability.

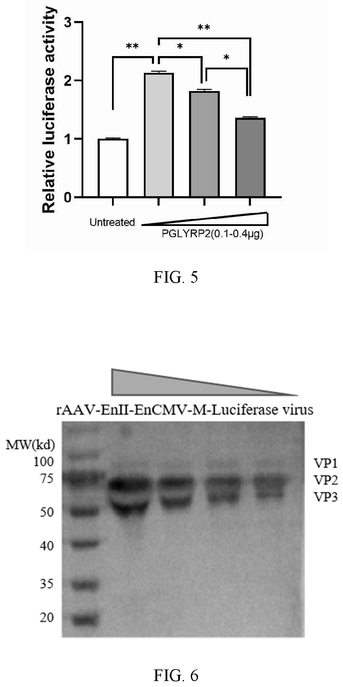

On the basis of the backbone vector pAAV-EnCMV-M, the negative regulatory element EnII of the PGLYRP2 protein was introduced to form the complete gene therapy delivery vector pAAV-EnII-EnCMV-M. Expression analysis in hepatocytes showed that under conditions of low PGLYRP2 protein expression, the PGLYRP2 protein predominantly exerted a positive regulatory effect on the expression of the gene therapy delivery vector pAAV-EnII-EnCMV-M. In contrast, under conditions of high PGLYRP2 protein expression, due to its inhibitory effect on EnII, the protein expression level of the gene therapy delivery vector was significantly reduced, indicating a negative regulatory effect on the gene therapy delivery vector. Therefore, the PGLYRP2 protein exerts a bidirectional feedback regulatory effect on the gene therapy delivery vector pAAV-EnII-EnCMV-M. Given that the bidirectional regulatory mechanism arises from the interplay among the PGLYRP2 protein, the PGLYRP2 promoter M, and the PGLYRP2 regulatory element, it is referred to as a self-feedback regulatory effect (as shown in FIG. 5).

### Example 3: Viral packaging, viral titer determination, and silver staining analysis of viral proteins for the gene therapy delivery vector rAAV-EnII-EnCMV-M-Luciferase

### (1) Viral packaging of the gene therapy delivery vector rAAV-EnII-EnCMV-M-Luciferase

HEK293T cells were seeded one day in advance. At the time of packaging, the cells reached 85% to 90% confluence, with uniform distribution and good condition. One hour before packaging, the culture medium was exchanged with serum-free DMEM (supplemented with 1% HEPES and 1% P/S). During medium exchange, the pipette tip was placed close to, but not touching, the inner wall of the culture dish. DMEM was slowly added to avoid detaching the adherent cells. Additionally, care was taken to avoid contaminating the medium and cells for medium exchange with the pipette tip. DMEM was added to a centrifuge tube, followed by the addition of plasmids pHelper, RC8, and pAAV-EnII-EnCMV-M-Luciferase at a molar ratio of 1:1:1 and the transfection reagent PEI (pHelper and RC8 were purchased from Addgene, with Cat. Nos. 112867 and 112864, respectively). The mixture was vortexed to mix thoroughly, and allowed to stand at room temperature for 30 minutes. An equal volume of the settled mixture was added to the HEK293T cells and recorded. The mixture was also slowly added along the wall to avoid detaching the cells, followed by gentle mixing (in a horizontal cross pattern). The cells were incubated in an incubator at 37°C with 5% CO₂. After 10 hours, the medium was exchanged. After 72 hours, the virus-producing cells along with the medium were collected into a 15 mL centrifuge tube. During collection, the culture dish was tilted at an angle and the cells were scraped into the medium. The mixture was centrifuged at 1000 rpm for 3 minutes to separate the cells and supernatant. The supernatant was stored separately, and the cell pellet was resuspended in 1 mL of PBS. The cell suspension was subjected to three freeze-thaw cycles between -80°C (an ultra-low temperature freezer) and room temperature, with vortexing after each thaw. The lysate was centrifuged at 8000 rpm for 1 hour. The supernatant was discarded, and the viral pellet was dissolved in an appropriate volume of PBS. After complete dissolution, the mixture was filtered through a 0.45 µm filter, and the filtrate was collected as concentrated AAV.

15%, 25%, 40%, and 60% iodixanol layers were prepared according to Table 1 below. Different concentrations of iodixanol were sequentially added to an ultracentrifuge tube (BECKMAN, Cat. No. 331372). Specifically, 4.2 mL of 60% iodixanol layer was first added, followed by the addition of 5 mL of 40% iodixanol layer, 6 mL of 25% iodixanol layer, and 9 mL of 15% iodixanol layer. The crude virus solution was carefully and slowly added to the top layer, and the tube cap was securely fastened. The ultracentrifuge tube was balanced with a weight difference controlled within 0.02 g, followed by centrifugation in an SW41Ti rotor at 36,300 rpm for 3 hours at 16°C.

**Table 1: Preparation of iodixanol layers of different concentrations and volume per tube**

| Layer | 60% iodixanol (mL) | 5 M NaCl (mL) | H₂O (mL) | 2.5 M KCl (µL) | 1 M MgCl (µL) | 10**×** PBS (mL) | Total (mL) | Volume per tube (mL) |
|---|---|---|---|---|---|---|---|---|
| 15% | 10 | 8 | 18 | 40 | 40 | 4 | 40 | 3.6 |
| 25% | 16.7 | 0 | 19.3 | 40 | 40 | 4 | 40 | 2.4 |
| 40% | 26.7 | 0 | 9.3 | 40 | 40 | 4 | 40 | 2 |
| 60% | 40 | 0 | 0 | 40 | 40 | 0 | 40 | 2 |

A syringe needle was inserted vertically through the outer wall of the ultracentrifuge tube. The visible "white band" between the 40% and 60% iodixanol layers was aspirated and transferred to a clean 50 mL centrifuge tube kept on ice. Since there is a possibility of contamination when precipitating viruses from the supernatant with PEG8000, the mixture was diluted with PBS + 0.001% PF68 to 15 mL, and filtered through a 0.45 µm filter membrane for sterilization. The filtrate was transferred to a 15 mL ultrafiltration tube (100 KDa) and centrifuged at 3500 rcf for 30 minutes at 4°C. The remaining liquid in the ultrafiltration tube was repeatedly pipetted and transferred to a virus storage tube. A certain volume of PBS + 0.001% PF68 was added to the concentration tube, mixed thoroughly by repeated pipetting, and the mixture was aspirated. Typically, after AAV centrifugal concentration, the remaining liquid was aspirated and washed twice with PBS + 0.001% PF68 (approximately 150 µL each wash). The virus solution was aliquoted in 50 µL portions into 200 µL EP tubes, and the tube walls were labeled with the virus name and date.

### (2) Viral titer determination

5 µL of virus solution was used for viral titer determination. Specifically, 5 µL of virus stock solution was added to 45 µL of ddH₂O to obtain a 10-fold diluted AAV, designated as AAV1; 5 µL of the 10-fold diluted virus solution was added to 45 µL of ddH₂O to obtain a 100-fold diluted AAV, designated as AAV2; 5 µL of the 100-fold diluted virus solution was added to 45 µL of ddH₂O to obtain a 1000-fold diluted AAV, designated as AAV3. The DNA concentration of pAAV-EnCMV-M-Luciferase was measured, and the copy number per µL of pAAV-EnCMV-M-Luciferase was calculated using the following formula. According to the formula, the copy number per µL of pAAV-EnCMV-M-Luciferase was calculated to be 7.8 × 10¹⁰ copies/µL.$\left(6.02\times {10}^{23}\right) \times \left(ng/μL\times {10}^{- 9}\right) / \left(DNA length\times 660\right) = copies / μL$

2 µL of pAAV-EnCMV-M-Luciferase stock solution was added to 18 µL of ddH₂O to obtain a plasmid dilution at approximately 10⁹ copies/µL. Serial dilutions ranging from 10³ to 10⁹ copies/µL were prepared in the same manner for later use.

Viral titer was determined by absolute quantitative PCR. Virus solutions and plasmids at various dilutions were used as templates for real-time quantitative PCR. The primers used for PCR were as follows: forward primer 5'-GATGAGCACTTTTAAAGTTCT-3' and reverse primer 5'-GTTGTCAGAAGTAAGTTGG-3'. The reaction system consisted of 5 µL of 2× traSYBR mixture (ComWin Biotech, CW0655M), 1 µL of a 10 µM mixture of forward and reverse primers, 2 µL of deionized water, and 2 µL of template. The PCR program was set according to the instructions of the ABI high-throughput quantitative PCR system (VIIA7), and the sample names as well as the names of the target and reference genes were configured. The program was run and the results were exported. A standard curve was generated based on the Ct values and copy number of plasmids at various dilutions, and the copy number of virus solution was calculated from the Ct values of viral samples.

### (3) Silver staining analysis of viral proteins for rAAV-EnII-EnCMV-M-Luciferase

12% stacking gel was prepared according to the following formulation: sodium dodecyl sulfate (SDS), 30% acrylamide (mass ratio of acrylamide: bis-acrylamide = 29:1), tetramethylethylenediamine (TEMED), Tris-HCl stacking buffer, ddH₂O, and ammonium persulfate (APS). 10% resolving gel was prepared according to the following formulation: SDS, 30% acrylamide (mass ratio of acrylamide: bis-acrylamide = 29:1), TEMED, Tris-HCl resolving buffer, ddH₂O, and APS. Viral samples at various dilutions were subjected to silver staining (according to the kit instructions, Biosharp, BL620A).

Results: A standard curve was plotted, and the copy number of virus solution was calculated to be 3.3 × 10¹¹ copies/mL. The silver staining results of viral proteins are shown in FIG. 6. The bands corresponding to VP1, VP2, and VP3 proteins were clearly visible, indicating successful packaging of recombinant adeno-associated virus.

### Example 4: Evaluation of specificity and long-lasting expression of rAAV-EnII-EnCMV-M-Luciferase virus

HEK293T cells and Huh7 cells were seeded into a 24-well cell culture plate. When the cells reached 50% to 70% confluence (corresponding to 2.5 × 10⁵ to 3.5 × 10⁵ cells per well), cell counting was performed. After aspirating the culture medium, the cells were washed with medium without fetal bovine serum. The cells in reserved wells were digested with trypsin, and digestion was terminated followed by cell counting. The required volume of rAAV to be added was calculated based on a multiplicity of infection (MOI, which refers to the number of viral particles per cell) of 10⁵. That is, 2.5 to 3.5 µL of virus solution was mixed with 250 µL of culture medium in a sterile 1.5 mL centrifuge tube. The virus-medium mixture was then added to the corresponding wells containing different cells. After addition of the virus mixture, the 24-well plate was incubated in a cell incubator at 37°C with 5% CO₂. After 2 hours, 250 µL of complete medium was added to each well, and the plate was returned to the incubator. After 12 hours, the medium was aspirated from the 24-well plate, and 500 µL of fresh complete medium was added to each well. Cell status was observed daily under a microscope. 60 hours after adeno-associated virus infection, the supernatant and cells were collected for luciferase reporter gene assays.

C57BL/6J mice were selected and injected via the tail vein with rAAV-EnII-EnCMV-M-Luciferase virus at a dose of 1 × 10¹¹ copies per mouse. 10 µL of virus solution was diluted with PBS to a final volume of 100 µL. After tail vein injection, the distribution of viral expression in mice at different time points was monitored using a PE IVIS Spectrum small animal *in vivo* imaging system. For small animal *in vivo* imaging, a luciferin stock solution at 15 mg/mL was prepared in sterile DPBS (without Mg²⁺ and Ca²⁺), mixed thoroughly, and filtered through a 0.2 µm filter membrane for sterilization. The mixture was used immediately or aliquoted and stored at -20°C protected from light, avoiding repeated freeze-thaw cycles. Luciferin was injected at a dose of 150 mg/kg body weight. Imaging analysis was performed 10 to 15 minutes after injection (when the luminescence signal reached the most stable plateau).

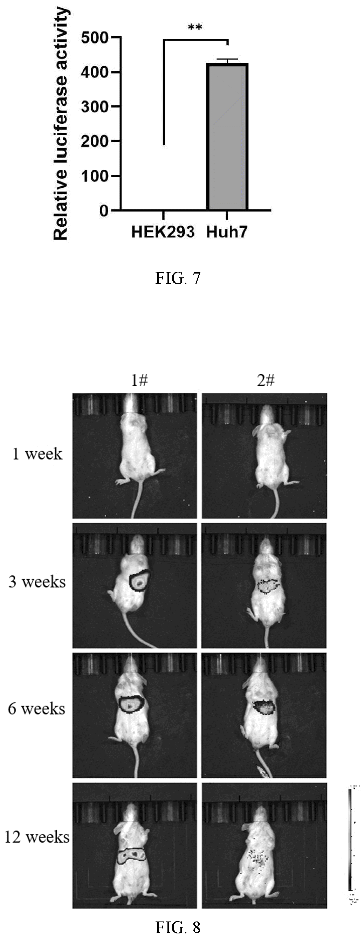

Results: The luciferase intensity of different cells infected with rAAV-EnII-EnCMV-M-Luciferase virus is shown in FIG. 7. The luciferase intensity in Huh7 cells was significantly higher than that in HEK293T cells (*p* < 0.001), indicating that the packaged rAAV-EnII-EnCMV-M-Luciferase virus exhibits good hepatocyte specificity. The *in vivo* imaging results of mice injected with the virus via the tail vein are shown in FIG. 8, where mouse 1# was injected with 1 × 10¹¹ copies of AAV and mouse 2# was injected with 0.5 × 10¹¹ copies of AAV. It can be seen from the *in vivo* imaging results that the packaged rAAV-EnII-EnCMV-M-Luciferase virus shows effective liver targeting and long-lasting expression in mice.

### Example 5: Identification of the nuclear localization signal functional domain of the PGLYRP2 protein

Confocal microscopy was used to observe the nuclear localization of the PGLYRP2 protein in the stably transfected cell line Huh7/PGLYRP2. Bioinformatic analysis was then performed to predict the nuclear localization signal (NLS) of the PGLYRP2 protein. The predicted NLS was fused to two red fluorescent proteins (2*RFP), and confocal microscopy was used to observe the subcellular localization of the 2*RFP-NLS fusion protein, thereby identifying the function of the NLS domain of the PGLYRP2 protein. The nucleotide sequence of the encoding gene of the NLS functional domain of the PGLYRP2 protein is shown in SEQ ID NO: 4 in the sequence listing.

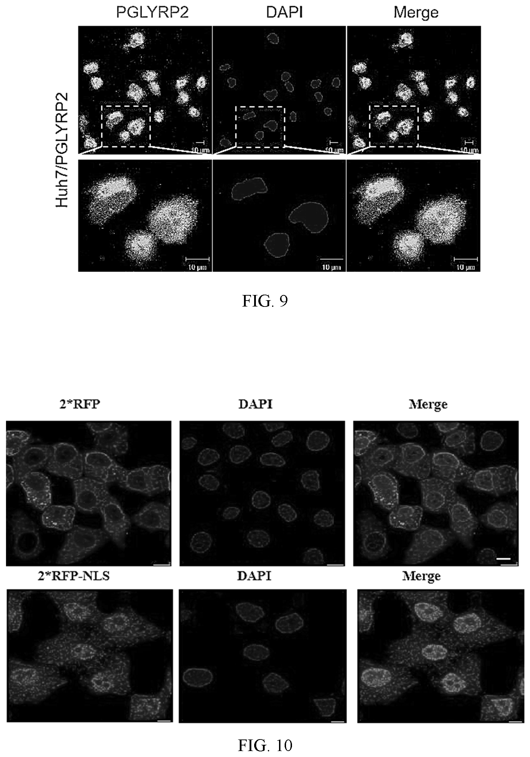

Results: In the stably transfected hepatocyte cell line Huh7/PGLYRP2, confocal microscopy revealed that the PGLYRP2 protein was predominantly localized in the nucleus (FIG. 9). Bioinformatic prediction indicated that the NLS of the PGLYRP2 protein is located at amino acids 550 to 576 at the C-terminus of the PGLYRP2 protein. Further analysis of the nuclear localization function of NLS by confocal microscopy revealed that the 2*RFP protein was predominantly localized in the cytoplasm, while the 2*RFP-NLS fusion protein was predominantly localized in the nucleus (FIG. 10). These findings clearly confirm that amino acids 550 to 576 at the C-terminus of the PGLYRP2 protein constitute the NLS functional domain of the PGLYRP2 protein.

### Example 6: Identification of the secretory signal peptide functional domain of the PGLYRP2 protein

Bioinformatic analysis was performed to predict the secretory signal peptide (SP) functional domain of the PGLYRP2 protein. The predicted SP was then fused to the green fluorescent protein EGFP-3*Flag to generate an SP-EGFP-3*Flag fusion protein. Confocal microscopy was used to analyze the intracellular green fluorescence levels in HEK293 cells transfected with pAAV-SP-EGFP-3*Flag and pAAV-EGFP-3*Flag. Immunoprecipitation (Flag pull-down) assays were performed to analyze the levels of the SP-EGFP-3*Flag fusion protein in the cell culture supernatant of HEK293 cells transfected with the two plasmids. The nucleotide sequence of the encoding gene of the secretory signal peptide functional domain of the PGLYRP2 protein is shown in SEQ ID NO: 6 in the sequence listing.

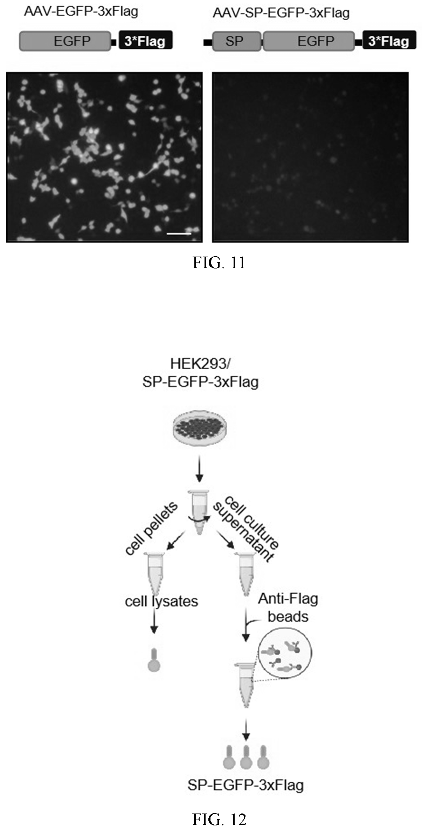

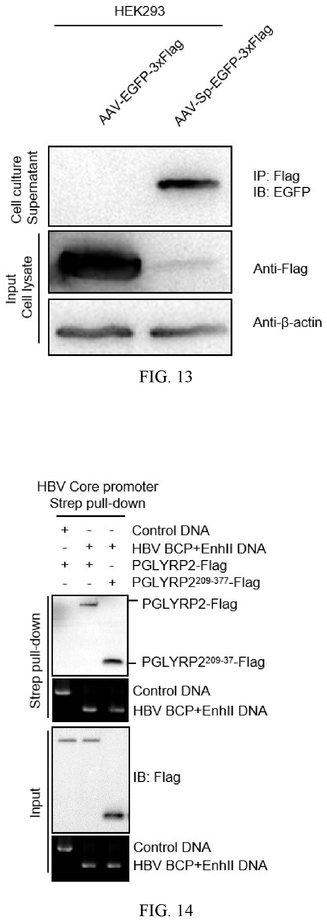

Results: In HEK293 cells transfected with the two plasmids, confocal microscopy revealed that the intracellular green fluorescence levels in the pAAV-EGFP-3*Flag group was higher than those in the pAAV-SP-EGFP-3*Flag group (FIG. 11), indicating that SP-EGFP-3*Flag may be secreted into the cell culture supernatant. The workflow of the Flag pull-down assay is shown in FIG. 12. The results demonstrated that the levels of the SP-EGFP-3*Flag fusion protein in the cell culture supernatant of HEK293 cells transfected with the two plasmids was significantly higher than those of the EGFP-3*Flag fusion protein (FIG. 13), further confirming the function of the secretory signal peptide (SP) functional domain of the PGLYRP2 protein.

### Example 7: Identification of the HBV DNA-binding domain of the PGLYRP2 protein

An HBV core promoter DNA-strep pull-down assay was used to identify the HBV DNA-binding domain of PGLYRP2. Subsequently, an HBV promoter-driven luciferase reporter gene assay system was used to analyze the HBV replication inhibitory function of the HBV DNA-binding domain PGLYRP2²⁰⁹⁻³⁷⁷ of PGLYRP2. The nucleotide sequence of the encoding gene of the HBV DNA-binding domain of the PGLYRP2 protein is shown in SEQ ID NO: 5 in the sequence listing.

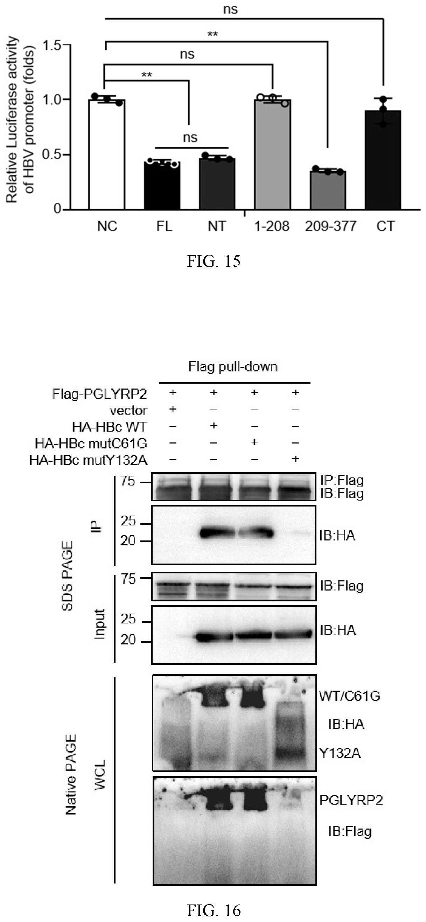

Results: The HBV core promoter DNA-strep pull-down assay demonstrated that the PGLYRP2 protein and its truncated form PGLYRP2²⁰⁹⁻³⁷⁷ were able to recognize and bind to the HBV core promoter DNA (FIG. 14), indicating that the functional domain PGLYRP2²⁰⁹⁻³⁷⁷ is the HBV DNA-binding domain of the PGLYRP2 protein. Further analysis of the regulatory effect of the PGLYRP2 protein and its truncated form on HBV replication using the HBV promoter-driven luciferase reporter gene assay system revealed that the HBV DNA-binding domain PGLYRP2²⁰⁹⁻³⁷⁷ of the PGLYRP2 protein exerted a clear inhibitory effect on HBV replication (FIG. 15).

### Example 8: Identification of the HBV nucleocapsid-binding functional domain of the PGLYRP2 protein

Method: HEK293 cells were co-transfected with expression plasmids encoding PGLYRP2-Flag and HA-tagged wild-type (WT) HBc or its mutants HA-HBc mutC61G (which preferentially forms capsids) and HA-HBc mutY132A (which forms HBc hexamers rather than capsids). Co-immunoprecipitation and native polyacrylamide gel electrophoresis were used to identify the binding of the PGLYRP2 protein to the HBV nucleocapsid. HEK293 cells were further co-transfected with expression plasmids encoding HA-HBc and PGLYRP2-Flag or its truncated form PGLYRP2^{PGRP}-Flag. Co-immunoprecipitation was used to analyze the interaction between the PGLYRP2 protein or its truncated form PGLYRP2^{PGRP} and the HBV core antigen HBc. The nucleotide sequence of the encoding gene of the HBV nucleocapsid-binding functional domain of the PGLYRP2 protein is shown in SEQ ID NO: 7 in the sequence listing.

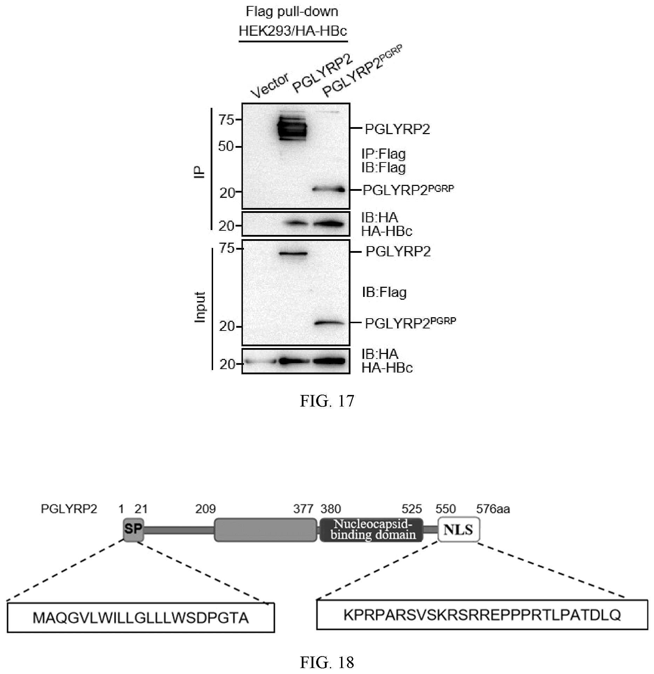

Results: Analysis by co-immunoprecipitation and native polyacrylamide gel electrophoresis revealed that the PGLYRP2 protein was able to bind to HA-HBc WT and the capsid-forming mutant HA-HBc mutC61G, but not to the non-capsid-forming mutant HA-HBc mutY132A (FIG. 16), indicating that the binding of the PGLYRP2 protein to the HBV core antigen HBc is HBV nucleocapsid-dependent. Further analysis of the interaction between the PGLYRP2 protein or its truncated form PGLYRP2^{PGRP} and the HBV core antigen HBc by co-immunoprecipitation revealed that the functional domain PGLYRP2^{PGRP} of the PGLYRP2 protein is the HBV nucleocapsid-binding functional domain (FIG. 17).

### Example 9: Construction of the adeno-associated virus gene therapy vector

The nuclear localization signal (NLS) and secretory signal peptide (SP) of the PGLYRP2 protein can be used to localize the functional domains of PGLYRP2 in the nucleus and extracellular environment, respectively. Specifically, fusion of the NLS with the HBV DNA-binding domain PGLYRP2²⁰⁹⁻³⁷⁷ facilitates the binding of the fusion protein PGLYRP2^{209-377aa}-NLS to HBV DNA and enhances its inhibitory effect on HBV replication; fusion of the SP with the HBV nucleocapsid-binding functional domain PGLYRP2^{PGRP} facilitates the inhibition of the assembly of the HBV nucleocapsid with HBV nucleic acids by the fusion protein SP-PGLYRP2^{PGRP}. The vector pLVSIN-PGLYRP2 used for amplification of the four functional domain genes of PGLYRP2 has been disclosed in the article Hepatology. 2020 May; 71(5): 1626-1642. A schematic diagram of the gene therapy delivery vector pAAV-EnII-EnCMV-M is shown in FIG. 1.

The gene therapy delivery vector pAAV-EnII-EnCMV-M was double-digested with restriction enzymes ClaI and BamHI, and ligated with the PGLYRP2^{209-377aa}-NLS-3flag fragment, followed by transformation to obtain a recombinant plasmid pAAV-EnII-EnCMV-M-PGLYRP2^{209-377aa}-NLS-3flag.

The PGLYRP2^{209-377aa}-NLS-3flag fragment was amplified using ClaI-PGLYRP2^{209-377aa}-NLS-3flag F and BamHI-PGLYRP2^{209-377aa}-NLS-3flag R as primers and the vector pLVSIN-PGLYRP2 as the template, wherein the forward primer ClaI-PGLYRP2^{209-377aa}-NLS-3flag F was CCATCGATGGATGAAATCCCCCCCTACCAT; the reverse primer BamHI-PGLYRP2^{209-377aa}-NLS-3flag R was CGGGATCCCGTTACTTGTCATCGTCATCCTTG.

The recombinant plasmid pAAV-EnII-EnCMV-M-PGLYRP2^{209-377aa}-NLS-3flag was double-digested with restriction enzymes BamHI and SalI, and ligated with an IRES fragment, followed by transformation to obtain a recombinant plasmid pAAV-EnII-EnCMV-M-PGLYRP2^{209-377aa}-NLS-3flag-IRES.

The IRES fragment was amplified using BamHI-IRES F and SalI-IRES R as primers and the plasmid pIRES (purchased from MiaoLing Plasmid Platform, Cat. No. P0786) as the template, wherein the forward primer BamHI-IRES F was CGGGATCCCGGTAAGTATCAAGGTTACAAGACA; the reverse primer SalI-IRES R was ACGCGTCGACGTCGGCCATAGCGGCCGCGGAATTATCATCGTGTTTTTCAAAGG; the nucleotide sequence of the IRES fragment is shown in SEQ ID NO: 8 in the sequence listing.

The recombinant plasmid pAAV-EnII-EnCMV-M-PGLYRP2^{209-377aa}-NLS-3flag-IRES was double-digested with restriction enzymes SalI and BglII, and ligated with an SP-PGLYRP2^{PGRP}-3flag fragment, followed by transformation to obtain a recombinant plasmid pAAV-EnII-EnCMV-M-PGLYRP2^{209-377aa}-NLS-IRES-SP-PGLYRP2^{PGRP}, which was the adeno-associated virus gene therapy vector.

The SP-PGLYRP2^{PGRP}-3flag fragment was amplified using SalI-SP-PGLYRP2^{PGRP}-3flag F and BglII-SP-PGLYRP2^{PGRP}-3flag R as primers and the vector pLVSIN-PGLYRP2 as the template, wherein the forward primer SalI-SP-PGLYRP2^{PGRP}-3flag F was ACGCGTCGACGTCGGCCATAGCGGCCGCGGAAATGGCCCAGGGCGTGCTCTGGAT T; the reverse primer BglII-SP-PGLYRP2^{PGRP}-3flag R was GAAGATCTTCTTACTTGTCATCGTCATCCTTG.

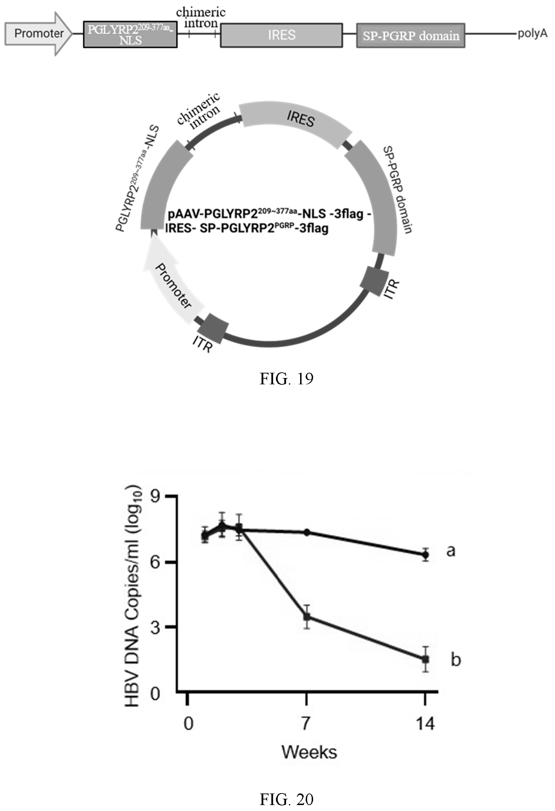

Results: A schematic diagram of the four functional domains of PGLYRP2 identified in the present disclosure is shown in FIG. 18. A schematic diagram of the HBV gene therapy recombinant plasmid pAAV-EnII-EnCMV-M-PGLYRP2^{209-377aa}-NLS-IRES-SP-PGLYRP2^{PGRP} is shown in FIG. 19.

### Example 10: Analysis of expression and viral clearance effect of the adeno-associated virus gene therapy vector in mouse liver tissue

C57BL/6J mice were injected via the tail vein with 1 × 10¹¹ copies of AAV/1.2*HBV to establish an infection model. Four weeks after modeling, HBV-positive mice were screened for subsequent gene therapy studies. Recombinant adeno-associated virus was then packaged with the adeno-associated virus gene therapy vector pAAV-EnII-EnCMV-M-PGLYRP2^{209-377aa}-NLS-IRES-SP-PGLYRP2^{PGRP} of the present disclosure, using the same viral packaging procedure as described in Example 3. The HBV gene therapy virus was injected via the tail vein into the HBV-positive C57BL/6J mice (1 × 10¹¹ copies per mouse). At 1, 2, 3, 7, and 14 weeks after virus injection, HBV viral titers (HBV DNA copy number) in the blood of mice were measured. At 14 weeks after virus injection, mouse livers were collected, and the expression level of the adeno-associated virus gene therapy vector was analyzed by immunoblotting. At 14 weeks after virus injection, mouse livers were collected, and extrachromosomal free DNA was extracted using the Hirt method. HBV cccDNA was then treated with ExoI/ExoIII/T5 exonuclease. The purified HBV cccDNA was quantified by real-time quantitative PCR. The primers used were cccDNA-specific primer_F: GTCTGTGCCTTCTCATCTGC and cccDNA-specific primer_R: ACAAGAGATGATTAGGCAGAGG. At 14 weeks after virus injection, blood was collected from mice by tail bleeding, and HBsAg levels in the serum were measured by ELISA (Sangon Biotech, D711407).

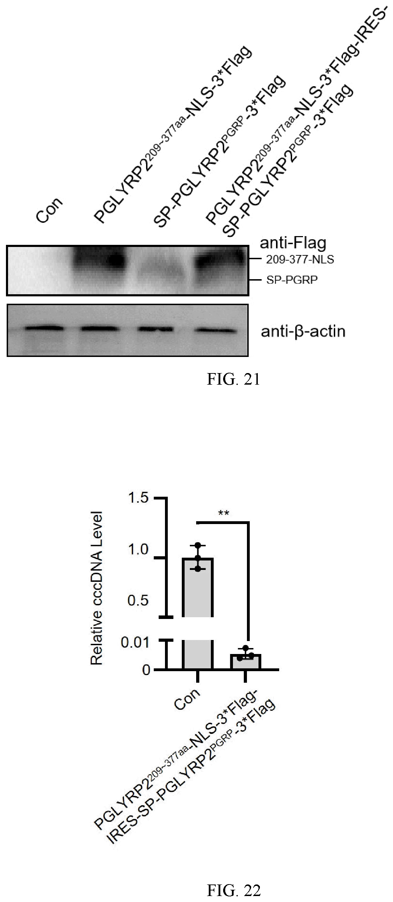

Results: The antiviral efficacy of the adeno-associated virus gene therapy vector was evaluated in the HBV mouse model. As shown in FIG. 20, HBV viral titers (HBV DNA copy number) in the blood of mice at 1, 2, 3, 7, and 14 weeks after virus injection are presented, where curve a represents the Con group and curve b represents the rAAV group. Compared with the control group, the HBV DNA copy number in the serum of mice treated with the adeno-associated virus gene therapy vector was extremely significantly reduced, indicating that the adeno-associated virus gene therapy vector of the present disclosure can significantly inhibit viral replication. At 14 weeks after virus injection, the expression of the target gene in the liver of mice is shown in FIG. 21. In the control group, the SP-fused SP-PGLYRP2^{PGRP} protein showed a relatively low intracellular level due to its extracellular secretion property, while the NLS-fused PGLYRP2^{209-377aa}-NLS protein showed a relatively high intracellular level. In the experimental group treated with the adeno-associated virus gene therapy vector expressing the dual-fusion protein, bands corresponding to both fusion proteins were visible.

C57BL/6J mice were injected with AAV at a dose of 1 × 10¹¹ copies. None of the 10 mice injected with the virus died or showed significant discomfort within 14 days, indicating that the adeno-associated virus gene therapy vector of the present disclosure has a safety profile.

At 14 weeks after virus injection, compared with the Con group (pAAV-CMV), the relative viral cccDNA content in the liver of mice in the rAAV group (pAAV-EnII-EnCMV-M-PGLYRP2^{209-377aa}-NLS-IRES-SP-PGLYRP2^{PGRP}) was reduced by approximately 200-fold, indicating that rAAV (pAAV-EnII-EnCMV-M-PGLYRP2^{209-377aa}-NLS-IRES-SP-PGLYRP2^{PGRP}) has a significant effect on eliminating the *in vivo* viral cccDNA pool (FIG. 22).

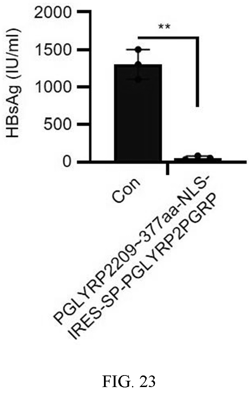

At 14 weeks after virus injection, compared with the Con group (pAAV-CMV), the viral HBsAg content in the blood of mice in the rAAV group (pAAV-EnII-EnCMV-M-PGLYRP2^{209-377aa}-NLS-IRES-SP-PGLYRP2^{PGRP}) was significantly reduced, indicating that rAAV (pAAV-EnII-EnCMV-M-PGLYRP2209-377aa-NLS-IRES-SP-PGLYRP2PGRP) has a significant effect on eliminating *in vivo* viral surface antigens (FIG. 23).

Current therapeutic strategies for chronic hepatitis B can effectively inhibit HBV replication but struggle to achieve a complete cure, because free cccDNA persists long-term in the nucleus of infected hepatocytes and cannot be eliminated by existing drugs, often leading to relapse after discontinuation of treatment. Therefore, the long-term persistence of cccDNA in infected hepatocytes is a key factor underlying chronic HBV infection and represents a major obstacle to curing hepatitis B. Drugs capable of eliminating the *in vivo* cccDNA pool will bring hope for a complete cure for hepatitis B.

Statistical analysis of the real-time quantitative PCR data for relative cccDNA content in mouse liver tissue at 14 weeks after virus injection showed that, based on an average turnover of 12 weeks in hepatocytes, the adeno-associated virus gene therapy vector of the present disclosure can significantly eliminate cccDNA within 14 weeks, indicating that the present disclosure has great potential to achieve a complete cure for hepatitis B.

## Claims

1. A viral gene therapy vector for eliminating hepatitis B virus, comprising a promoter, an intron, an enhancer, an encoding gene of the nuclear localization signal NLS functional domain of the PGLYRP2 protein, an encoding gene of the HBV DNA-binding domain of the PGLYRP2 protein, an encoding gene of the secretory signal peptide functional domain of the PGLYRP2 protein, an encoding gene of the HBV nucleocapsid-binding functional domain of the PGLYRP2 protein, and an IRES fragment.

2. The viral gene therapy vector for eliminating hepatitis B virus according to claim 1, wherein the promoter is a truncated promoter M of the PGLYRP2 gene, the nucleotide sequence of which is shown in SEQ ID NO: 1 in the sequence listing.

3. The viral gene therapy vector for eliminating hepatitis B virus according to claim 2, wherein the enhancer comprises a CMV enhancer and an HBV EnII element, wherein the nucleotide sequence of the CMV enhancer is shown in SEQ ID NO: 2 in the sequence listing, and the nucleotide sequence of the HBV EnII element is shown in SEQ ID NO: 3 in the sequence listing.

4. The viral gene therapy vector for eliminating hepatitis B virus according to claim 3, wherein the encoding gene of the nuclear localization signal NLS functional domain of the PGLYRP2 protein is a gene encoding amino acids 550 to 576 at the C-terminus of the PGLYRP2 protein, the nucleotide sequence of which is shown in SEQ ID NO: 4 in the sequence listing.

5. The viral gene therapy vector for eliminating hepatitis B virus according to claim 4, wherein the nucleotide sequence of the encoding gene of the HBV DNA-binding domain of the PGLYRP2 protein is shown in SEQ ID NO: 5 in the sequence listing.

6. The viral gene therapy vector for eliminating hepatitis B virus according to claim 5, wherein the nucleotide sequence of the encoding gene of the secretory signal peptide functional domain of the PGLYRP2 protein is shown in SEQ ID NO: 6 in the sequence listing.

7. The viral gene therapy vector for eliminating hepatitis B virus according to claim 5, wherein the nucleotide sequence of the encoding gene of the HBV nucleocapsid-binding functional domain of the PGLYRP2 protein is shown in SEQ ID NO: 7 in the sequence listing.

8. The viral gene therapy vector for eliminating hepatitis B virus according to claim 1, wherein the vector backbone of the viral gene therapy vector is an adeno-associated viral vector, an adenoviral vector, a lentiviral vector, or a retroviral vector; the adeno-associated viral vector is an AAV2, AAV5, AAV7, AAV8, AAV9, or AAV-DJ adeno-associated viral vector.

9. A medicament for treating hepatitis B virus infection, comprising the viral gene therapy vector according to any one of claims 1 to 8.

10. Use of the viral gene therapy vector according to any one of claims 1 to 8 in the manufacture of a medicament for treating hepatitis B virus infection.
